# EUROPÄISCHE PATENTSCHRIFT

(11) **EP 2 007 457 B1**
(45) Veröffentlichungstag und Bekanntmachung des Hinweises auf die Patenterteilung: **14.02.2018**
(21) Anmeldenummer: 07727795.2
(22) Anmeldetag: 04.04.2007
(51) Int. Cl.: A61M 15/00

(54) **INHALATOR**
INHALER
INHALATEUR

(30) Priorität: 11.04.2006 DE 102006016904
(43) Veröffentlichungstag der Anmeldung: 31.12.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: WALZ, Michael, 55411 Bingen (DE)
(74) Vertreter: Simon, Elke Anna Maria
(86) Internationale Anmeldenummer: PCT/EP2007/053326
(87) Internationale Veröffentlichungsnummer: WO 2007/118801

(56) Entgegenhaltungen:
- EP-A1- 0 388 621
- EP-A2- 0 696 458
- WO-A-02/24268
- WO-A-2004/047796
- WO-A-2004/062716
- WO-A1-02/074374
- WO-A2-2006/030210
- US-A- 5 685 294

## Beschreibung

Die Erfindung bezieht sich auf einen Inhalator, insbesondere Pulverinhalator, zur Verabreichung von einem Arzneimittel in Form inhalationsfähiger Substanzen, Substanzformulierungen oder -mischungen mit einem eine Kammer zur Aufnahme der Substanzen aufweisenden Gehäuse.

Die EP 0 911 047 A1 offenbart einen Inhalator für die Inhalation pulverförmiger Arzneimittel aus Kapseln, der ein Unterteil mit zwei Fenstern und eine Platte, in der sich Kapselhalterungen sowie Lufteinlassöffnungen befinden, umfasst. Im Weiteren ist eine Inhalationskammer mit der Platte verbunden, an der ein mit zwei geschliffenen Nadeln versehener, gegen eine Feder beweglicher Kopf vorgesehen ist. Ein Mundrohr ist mit einem Oberteil des Inhalators und ein Deckel klappbar mit dem Unterteil, der Platte sowie dem Oberteil verbunden. Als problematisch erweist es sich, dass der Inhalator zumindest einmal im Monat gereinigt werden sollte, um insbesondere Arzneimittelrückstände zu entfernen, da diese Rückstände zu regulatorischen Problemen führen können, wenn sie sich in unregelmäßigen Abständen lösen und mit der eigentlichen Dosis ausgebracht werden. Zur Reinigung ist der Deckel zu öffnen und das Mundstück sowie die Platte von dem Unterteil weg zu schwenken. Der komplette Inhalator wird anschließend mit vorzugsweise warmem Wasser ausgespült und an der Luft getrocknet. Weiterhin ist eine Reinigung durch das Auswischen des Inhalators mittels feuchten Zellstoffs bzw. Wattestäbchen erforderlich. Der verhältnismäßig einfach zu bewerkstelligende Reinigungsprozess wird jedoch in Abhängigkeit von der persönlichen Vorgehensweise der Benutzer des Inhalators, ihrem Alter und ihrem Erkrankungsgrad unterschiedlich konsequent durchgeführt. Des Weiteren ist die zuvor geschilderte Reinigung bei der Verwendung eines Arzneimittels mit einem niedrigen Wirkstoffanteil bzw. einem wasserlöslichen Wirkstoff in der Regel erfolgreich, während sie bei einem Arzneimittel mit einem hohen Wirkstoffanteil bzw. einem schlecht wasserlöslichen Wirk**stoff nur** schwer vorzunehmen ist.

Die US5685294A und die WO2004062716A zeigen Pulverinhalatoren mit einem Magazin von Kapselkammern, die jeweils mit einer Kapsel bestückt sind. Das Magazin kann komplett durch ein Magazin mit neuen Kapseln ersetzt oder alternativ können die Kapseln im Magazin ersetzt werden. Die WO2004062716A beschreibt zusätzlich die Mikrostrukturierung von inneren Oberflächen, die mit Pulveraerosol in Berührung kommen können (z.B, in Kapselkammern), wobei diese mikrostrukturierten Oberflächen besonders einfach gereinigt werden können. Die WO2006/030210 zeigt einen Pulverinhalator, zur Verabreichung von einem in einer Kapsel beinhalteten Arzneimittel, wobei der Pulverinhalator ein Gehäuse mit einer Kammer zur Aufnahme der Substanzen hat und die Kammer unlösbar mit einem Inhalationskanal verbunden und gemeinsam mit dem Inhalationskanal austauschbar ist. Die EP069645SA2 zeigt einen Inhalator zur Verabreichung feiner granularer Medizin, wobei der Inhalator ein zylindrisches Inhalierstück mit einer Aufnahme für einen zylindrischen Pulver-Halter bzw. für einen Kapselhalter in einer anderen Ausführungsform beinhaltet. Aufnahme und Halter weisen Nadel-Einführöffnungen auf, die in Überdeckung gebracht werden müssen, damit Nadeln zur Lochung der im Halter eingesetzten Kapsel eingebracht werden können. Durch die Lochung wird ein Durchgang für Luftströmung durch die Kapsel hindurch geöffnet. Inhalierstück und Kapselhalter sind voneinander trennbar, so dass die Reinigung des eine Inhalationsöffnung aufweisenden Hauptkörpers des Geräts möglich wird.

Die WO2002/024268 betrifft einen Pulverinhalator, in dem abgemessene Dosiseinheiten eines medizinischen Pulvers auf einer austauschbaren Kassette bereitgestellt werden, wobei innerhalb des Geräts sequentiell auf die Dosiseinheiten zugegriffen werden kann. Die Kassette weist eine mechanische Kodierung auf, so dass nur die richtige Kassettenart in die Öffnung eines Inhalators eingesetzt werden kann und so das Risiko, ein unerwünschtes Pulver ins Gerät einzuführen, unterbunden wird. Die einzelnen Dosiseinheiten sind mit einer Schutzfolie verschlossen, die aufgeschnitten wird, damit ein Saugrohr Zugang zum Pulver bekommt. Ein gemeinsamer Austausch von Mundstück und Saugrohr wird erwähnt.

Es ist Aufgabe der Erfindung, einen Inhalator der eingangs genannten Art zu schaffen, der für einen Patienten einfach handhabbar ist.

Erfindungsgemäß wird die Aufgabe dadurch gelöst, dass die Kammer austauschbar in dem Gehäuse angeordnet ist.

Aufgrund dieser Maßnahme ist es nicht erforderlich, den Inhalator mittels Wasser von Arzneimittelrückständen zu reinigen und die Gefahr einer Pulveragglomeration aufgrund einer unvollständigen Trocknung des Inhalators ist wesentlich reduziert.

Inhalatoren sind unter den Markennamen HandiHaler®, Spinhaler®, Rotahaler®, Aerolizer®, Flowcaps®, Turbospin®. AIR DPI®, Orbital®, Directhaler® bekannt und/oder in DE 33 45 722, EP 0 591 136, DE 43 18 455, WO 91/02558, FR-A-2 146 202, US-A-4 069 819, EP 666085, EP 869079, US 3,991,761, WO 99/45987, WO 200051672, Bell, J. Pharm. Sci. 60, 1559 (1971); Cox, Brit. Med. J. 2, 634 (1969), beschrieben. Als Pulverinhalatoren sind Einfach- oder Mehrfachdosis-Pulverinhalatoren, insbesondere der Spinhaler®, Rotahaler®, Aerolizer®, Inhalator®, HandiHaler®, Diskhaler®, Diskus®, Accuhaler®, Aerohaler®, Eclipse®, Turbohaler®, Turbuhaler®, Easyhaler®, Novolizer®, Clickhaler®, Pulvinal®, Novolizer®, SkyeHaler®, Xcelovair®, Pulvina,⁵, Taifun®, MAGhaler®, Twisthaler® und der Jethaler® bekannt.

In Ausgestaltung ist die Kammer zur Aufnahme einer das Arzneimittel aufnehmenden Kapsel ausgebildet, Die in die Kammer eingesetzte Kapsel bildet demnach die eigentliche Aufnahme für das Arzneimittel,

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmem, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl- 2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino }ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl} 4H-benzo[1,4]oxazin-3-on
- 6-Hydroxy-8-{1-hydroxy-2-[2-(4-phenoxy-essigsäureethylester)-1, 1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl} -4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{ 1-hydroxy-2-[2-(4-hydroxy-phenyl)-1,1-dimethyl-ethylamino]-ethyl}-4H-benzo[1,4]oxazin-3-on
- 6-Hydroxy-8- {1-hydroxy-2-[2-(4-isopropyl-phenyl)- 1, 1dimethyl-ethylamino}-4H-benzo[1,4]oxazin-3-on
- 8-{2-[2-(4-Ethyl-phenyl)-1,1-dimethyl-ethylaminol-1-hydroxy-ethyl}-6-hydroxy-4H-benzo[1,4]oxazin-3-on
- 8-{2-[2-(4-Ethoxy-phenyl)-1,1-dimethyl-ethylaminol-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino } -ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino }-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{ 6-[2-Hydroxy-2-(4-hydroxy-3-hydroxymethyl-phenyl)-ethylamino]-hexyloxy }-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl }-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 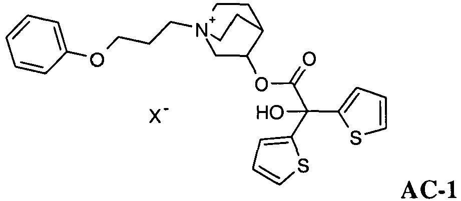 worin X ⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 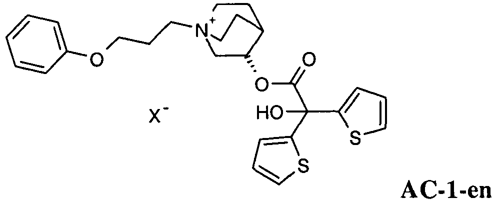 enthalten, worin X ⁻ die vorstehend genannten Bedeutungen aufweisen kann. Weiterhin bevorzugte Anticholinergika sind ausgewählt aus den Salzen der Formel AC-2 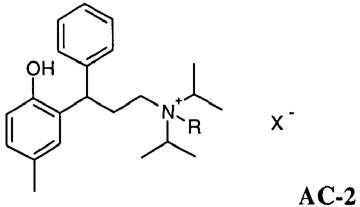 worin R entweder Methyl oder Ethyl bedeuten und worin X ⁻ die vorstehend genannte Bedeutungen aufweisen kann. In einer alternativen Ausführungsform kann die Verbindung der Formel **AC-2** auch in Form der freien Base **AC-2-base** vorliegen.

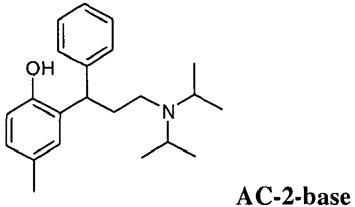

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, A-riflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*, 10*b*S*)-9-Ethoxy-1,2,3,4,4a, 10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)- 2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6- {[4-(N,N-diethylamino)-1-oxo-2-buten-1-yl]-amino }-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino }-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino }-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{ [3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy} -7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan- 1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)aminol-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy }-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{ 1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino }-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-[2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Für die Inhalation kommen Arzneimittel mit den o.g. Wirkstoffen in Betracht, sowie deren Salze, Ester sowie die Kombination dieser Wirkstoffe, Salze und Ester.

Bevorzugt ist die Kammer in einer Kammeraufnahme einer schwenkbar an dem Gehäuse angelenkten sowie mit dem Gehäuse verrastbaren Platte angeordnet. Demnach wird die Kammer zunächst mit der an der Platte angeordneten Kammeraufnahme gekoppelt und anschließend die Platte mit der Kammeraufnahme in eine Gebrauchsposition verschwenkt, in der die Platte mit dem Gehäuse verrastet ist und dasselbe verschließt. Es sind Mittel vorgesehen, die die Kammer in einer definierten Lage halten. Die Mittel fixieren die Kammer gegenüber einem Mundstück. Die Mittel sind als eine Nut-Feder-Verbindung ausgestaltet. Z.B. ist es möglich, dass die Kammeraufnahme eine Nut und die Kammer eine dazu korrespondierende Feder, oder umgekehrt, aufweist, wobei die Nut beim Einsetzen der Kammer in die Kammeraufnahme die Feder übergreift und damit die beiden Bauteile zueinander gehalten sind. Im Weiteren ist es möglich, die Nut und die Feder derart konisch zu gestalten, dass bei einer bestimmten Einschubtiefe eine Klemmwirkung eintritt. Bevorzugt umfassen die Mittel eine Steck-Kodierung. Damit ist ein verdrehtes Einsetzen der Kammer in die Kammeraufnahme ausgeschlossen. Vorteilhafterweise ist in der Kammeraufnahme ein Anschlag für die eingeschobene Endlage der Kammer vorgesehen.

Nach einer Weiterbildung ist die Kammer im Querschnitt im Wesentlichen zylindrisch ausgebildet, wobei die in Richtung eines Mundstückes weisende Stirnseite mit einem zu dem Mundstück führenden Inhalationskanal verbunden ist und die dem Mundstück gegenüberliegende Stirnseite mit einer Lufteintrittsöffnung des Gehäuses in Wirkverbindung steht. In weiterer Ausgestaltung ist die Kammer unlösbar mit dem Inhalationskanal verbunden und gemeinsam mit dem Inhalationskanal austauschbar. Die einstückige Ausbildung der Kammer mit dem Inhalationskanal kann beispielsweise durch eine Fertigung im Kunststoffspritzgussverfahren erfolgen und gewährleistet den Austausch wesentlicher Bauteile des Inhalators, die mit dem Arzneimittel beim Inhalieren in Berührung kommen. Vorzugsweise handelt es sich bei den Kunststoffen um Polymerisate, thermoplastische Polykondensate, Polyaddukte, abgewandelte Naturstoffe oder Kautschuke bzw. um Gemische dieser Kunststoffe.

Besonders bevorzugt sind hier Polyolefine, Vinylchlorid-Polymerisate, StyrolPolymerisate, Polyacetale, Polyamide, thermoplastische Polyester und Polyarylether bzw. Gemische dieser Kunststoffe. Beispiele für diese Kunststoffe sind Polyethylen, Polyvinylchlorid, Polyoxymethylen, Polyacetal, Acrylnitril/Butadien/Styrol(ABS), Acrylnitril/Styrol/Acryl-ester(ASA), Polyamide, Polycarbonat, Poly (ethylenterephthalat), Poly(butylenterephthalat) oder Poly(phenylenether). Derartige Kunststoffe können beispielsweise von der Firma Ensinger in Deutschland, Nufringen, bezogen werden.

Zweckmäßigerweise ist in dem Inhalationskanal ein Bauteil, insbesondere ein Sieb, zum Dispergieren von Partikeln angeordnet. Demnach gelangt beim Inhalieren das insbesondere mikronisierte Arzneimittel durch das Sieb zu dem Benutzer des Inhalators.

Um beim Inhalieren eine hinreichende Durchströmung der Kammer mit Luft und damit den Austrag des Arzneimittels aus der Kapsel sowie die Förderung des Arzneimittels durch den Luftstrom sicherzustellen, weist die Kammer auf der dem Inhalationskanal gegenüberliegenden Stirnseite einen Stutzen zur Kopplung mit der Lufteintrittsöffnung des Gehäuses auf. Zur Entnahme der Kammer aus der Kammeraufnahme steht zweckmäßigerweise der Stutzen über die Kammeraufnahme vor. Durch eine Druckbeaufschlagung des Stutzens in axialer Richtung der Kammer lässt diese sich aus der Kammeraufnahme herausdrücken und entnehmen.

Damit die in der Kammer vorhandene Kapsel, die das Arzneimittel vor Umgebungseinflüssen schützt, mit einfachen Mitteln zu öffnen ist, weist die Kammer auf ihrem Umfang zwei zueinander beabstandete Bohrungen auf, in die Nadeln zum jeweils endseitigen Einstechen der Kapsel eingreifen, wobei die Nadeln in der Kammeraufnahme verschiebbar gelagert und mittels eines Betätigungselementes beaufschlagbar sind. Selbstverständlich ist es im Rahmen der Erfindung ebenfalls umfasst, lediglich eine in der Kammeraufnahme verschiebbar gelagerte und mittels des Betätigungselementes beaufschlagbare Nadel zum Einstechen der Kapsel vorzusehen.

Vorzugsweise ist die Kammer zur einmaligen Verwendung ausgebildet und enthält die Kapsel. Die Kapsel kann herstellerseitig in der Kammer unverlierbar bzw. nicht entnehmbar zum einmaligen Inhalieren untergebracht werden. Nach dem Inhalieren wird die Kammer mitsamt der entleerten Kapsel dem Inhalator entnommen und entsorgt. Dadurch steht bei jedem Inhalieren eine saubere Kammer, gegebenenfalls mit zugeordnetem Inhalationskanal sowie Stutzen, zur Verfügung.

Zur optischen Kontrolle, ob eine Kapsel in die Kammer eingesetzt bzw. das gesamte vorhandene Arzneimittel inhaliert wurde ist die Kammer aus einem transparenten Kunststoff gefertigt. Vorzugsweise weisen das Gehäuse und die Kammeraufnahme zumindest im Bereich der Kammer jeweils wenigstens einen aus einem transparenten Kunststoff gefertigten Bereich, insbesondere ein Fenster, auf.

Für die Realisierung eines im Aufbau kompakten und leicht zu transportierenden Inhalators sind vorteilhafterweise gemeinsam mit der Platte das Mundstück und ein Deckel an dem Gehäuse schwenkbar angelenkt, wobei das Mundstück mit der Platte und der Deckel mit dem Gehäuse verrastbar ist.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:

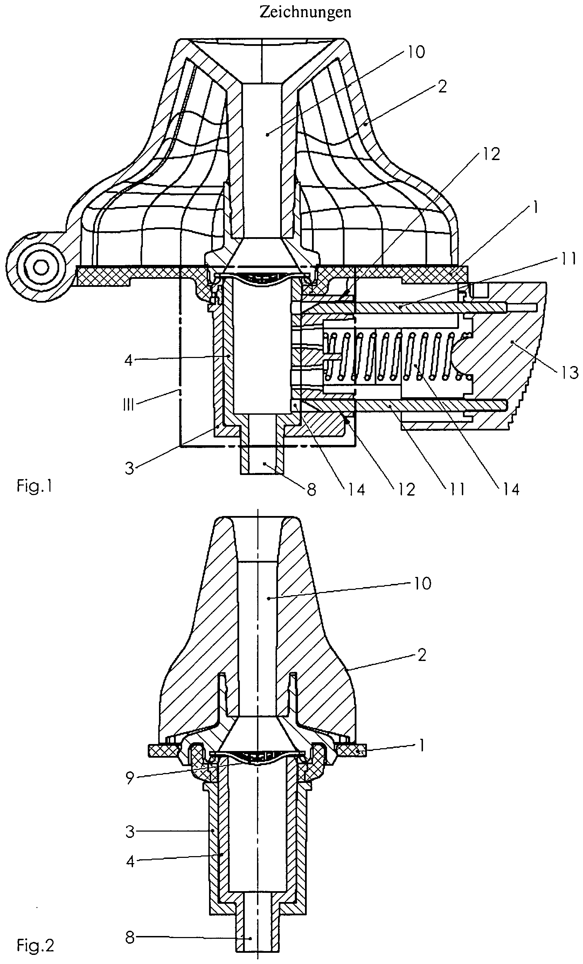

- Fig.1: eine Teildarstellung eines erfindungsgemäßen Inhalators,
- Fig.2: eine weitere Teildarstellung des Inhalators nach Fig. 1,

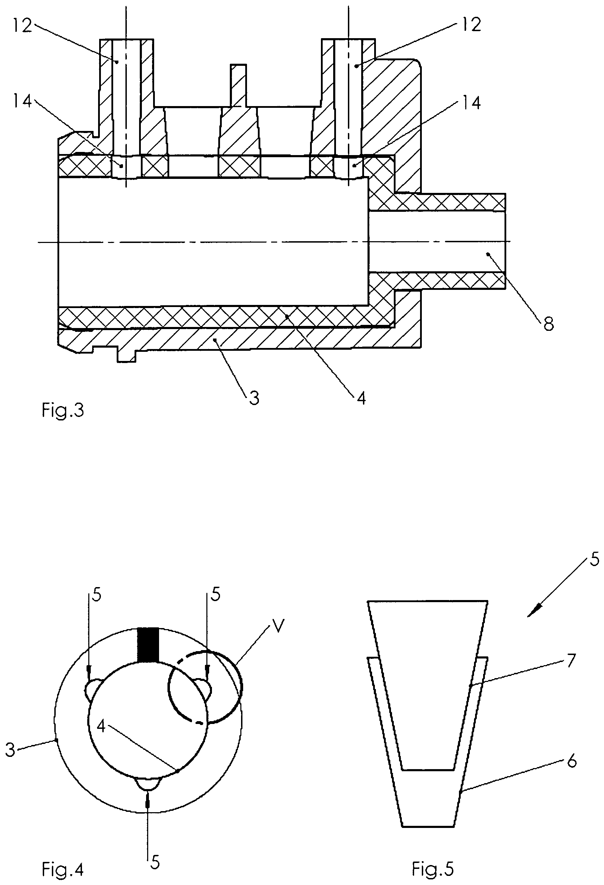

- Fig.3: eine vergrößerte Darstellung der Einzelheit III nach Fig. 1,
- Fig.4: eine schematische Darstellung eines Querschnittes durch die Einzelheit nach Fig. 3 und
- Fig.5: eine vergrößerte Seitenansicht der Einzelheit V nach Fig. 4,

Der Inhalator umfasst ein als Unterteil ausgebildetes Gehäuse, an dem eine Platte 1 verschwenkbar angelenkt ist, die in einer verrasteten Endlage das Gehäuse verschließt. Gemeinsam mit der Platte 1 sind ein Mundstück 2 und ein Deckel an dem Gehäuse gelagert, wobei das Mundstück 2 mit der Platte 1 und der Deckel mit dem Gehäuse verrastbar ist An der Platte 1 ist eine Kammeraufnahme 3 vorhanden, die zum auswechselbaren Einsetzen einer Kammer 4 zur Aufnahme einer ein Arzneimittel aufnehmenden Kapsel ausgebildet ist. Die Kammeraufnahme 3 ist derart an der Platte 2 befestigt, dass sie in das Gehäuse hinein und aus dem Gehäuse herausgeschwenkt werden kann. Um die Kammer 4 in einer definierten Lage zu der Kammeraufnahme 3 zu halten, ist eine Steck-Kodierung in Form von drei ungleichmäßig über den Umfang verteilten Nut-Feder-Verbindungen 5 vorgesehen, die im Weiteren sicherstellen, dass die Kammer 4 nur in einer bestimmten Lage in die Kammeraufnahme 3 eingesetzt werden kann. Die Nuten 6 und die darin eingreifenden Federn 7 sind derart konisch ausgeführt, dass sie sich von der Platte ausgehend verjüngen, um zum einen ein verhältnismäßig einfaches Zusammenfügen der Kammer 4 mit der Kammeraufnahme 3 und zum anderen zumindest in einer in die Kammeraufnahme 3 eingeschobenen Endlage der Kammer 3 eine Klemmwirkung zu erzielen.

Die Kammer 4 weist einen Stutzen 8 auf, durch den beim Inhalieren Luft in die Kammer 4 gelangt und der auf der dem Mundstück 2 abgewandten Seite über das freie Ende der Kammeraufnahme 3 hervorsteht. Bei einer manuellen Druckbeaufschlagung des Stutzens 8 seitens eines Benutzers wird die Kammer 4 aus der Kammeraufnahme 3 gedrückt, um sie auswechseln zu können. Auf der dem Mundstück 2 zugewandten Seite liegt die Kammer 4 an einem Sieb 9 an, das in einen Inhalationskanal 10 eingesetzt ist, um Partikeln zu dispergieren.

Für das Aufstechen der Kapsel unmittelbar vor dem Inhalieren sind zwei zueinander beabstandete Nadeln 11 in Führungsstutzen 12 der Kammeraufnahme 3 verschiebbar gelagert, denen ein gemeinsames Betätigungselement 13 zugeordnet ist. Die Führungsstutzen 12 der Kammeraufnahme 3 korrespondieren zu Bohrungen 14 auf dem Umfang der Kammer 4, durch die die Nadeln 11 in das Innere der Kammer 4 und damit in die Kapsel gelangen. Nachdem die Kapsel mittels der Nadeln 11 aufgestochen wurde, erfolgt durch eine zwischen der Kammeraufnahme 3 und dem Betätigungselement 13 angeordnete Druckfeder 14 eine Rückstellbewegung der Nadeln 11 zur Freigabe der Löcher in der Kapsel.

## Patentansprüche

1. Inhalator, insbesondere Pulverinhalator, zur Verabreichung von einem Arzneimittel in Form inhalationsfähiger Substanzen, Substanzformulierungen oder -mischungen mit einem Gehäuse, das eine Kammer (4) zur Aufnahme der Substanzen aufweist, wobei die Kammer (4) im Querschnitt im Wesentlichen zylindrisch ausgebildet ist, wobei die in Richtung eines Mundstückes (2) weisende Stirnseite mit einem zu dem Mundstück (2) führenden Inhalationskanal (10) verbunden ist und die dem Mundstück (2) gegenüberliegende Stirnseite mit einer Lufteintrittsöffnung des Gehäuses in Wirkverbindung steht, wobei die Kammer (4) austauschbar in dem Gehäuse angeordnet ist und die Kammer (4) unlösbar mit dem Inhalationskanal (10) verbunden und gemeinsam mit dem Inhalationskanal (10) austauschbar ist, wobei die Kammer (4) in einer Kammeraufnahme (3) angeordnet ist,
**dadurch gekennzeichnet, dass** die Kammer (4) mittels einer Nut-Feder-Verbindung (5) in der Kammeraufnahme (3) gegenüber dem Mundstück (2) fixiert ist.

2. Inhalator nach Anspruch 1, **dadurch gekennzeichnet, dass** die Kammer (4) zur Aufnahme einer das Arzneimittel aufnehmenden Kapsel ausgebildet ist.

3. Inhalator nach einem der Ansprüche 1 oder 2, **dadurch gekennzeichnet, dass** die Kammer (4) mittels einer Steck-Kodierung in einer definierten Lage gehalten wird.

4. Inhalator nach einem der Ansprüche 1 bis 3, **dadurch gekennzeichnet, dass** in der Kammeraufnahme (3) ein Anschlag für die eingeschobene Endlage der Kammer (4) vorgesehen ist.

5. Inhalator nach einem der Ansprüche 1 bis 4, **dadurch gekennzeichnet, dass** in dem Inhalationskanal (10) ein Bauteil, insbesondere ein Sieb (9), zum Dispergieren von Partikeln angeordnet ist.

6. Inhalator nach einem der Ansprüche 1 bis 5, **dadurch gekennzeichnet, dass** die Kammer (4) auf der dem Inhalationskanal (10) gegenüberliegenden Stirnseite einen Stutzen (8) zur Kopplung mit der Lufteintrittsöffnung des Gehäuses aufweist.

7. Inhalator nach Anspruch 2, **dadurch gekennzeichnet, dass** die Kammer (4) in einer Kammeraufnahme (3) angeordnet ist und die Kammer (4) auf ihrem Umfang zwei zueinander beabstandete Bohrungen (14) aufweist, in die Nadeln (11) zum jeweils endseitigen Einstechen der Kapsel eingreifen, wobei die Nadeln (11) in der Kammeraufnahme (3) verschiebbar gelagert und mittels eines Betätigungselementes (13) beaufschlagbar sind.

8. Inhalator nach Anspruch 2, **dadurch gekennzeichnet, dass** die Kammer (4) zur einmaligen Verwendung ausgebildet ist und die Kapsel enthält, wobei die Kapsel herstellerseitig in der Kammer (4) nicht entnehmbar untergebracht ist.

9. Inhalator nach einem der Ansprüche 1 bis 8, **dadurch gekennzeichnet, dass** die Kammer (4) in einer Kammeraufnahme (3) angeordnet ist und das Gehäuse und die Kammeraufnahme (3) zumindest im Bereich der Kammer (4) jeweils wenigstens einen aus einem transparenten Kunststoff gefertigten Bereich, insbesondere ein Fenster, aufweisen.

## Claims

1. Inhaler, in particular a dry powder inhaler, for administering a medicament in the form of inhalable substances, substance formulations or substance mixtures, comprising a casing having a chamber (4) for receiving the substances, the chamber (4) being substantially cylindrical in cross-section, the front side which faces towards a mouthpiece (2) being connected to an inhalation channel (10) leading to the mouthpiece (2), and the front side that is opposite the mouthpiece (2) being operatively connected to an air inlet opening in the casing, wherein the chamber (4) being arranged in the casing so as to be replaceable, and the chamber (4) being non-releasably connected to the inhalation channel (10) and being replaceable together with the inhalation channel (10), and the chamber (4) being arranged in a chamber receptacle (3), **characterised in that** the chamber (4) is fixed relative to the mouthpiece (2) in the chamber receptacle (3) by a tongue-and-groove joint (5).

2. Inhaler according to claim 1, **characterised in that** the chamber (4) is designed to receive a capsule holding the medicament.

3. Inhaler according to claim 1 or claim 2, **characterised in that** the chamber (4) is held in a defined position by means of a plug coding.

4. Inhaler according to any of claims 1 to 3, **characterised in that** an end stop for the inserted final position of the chamber (4) is provided in the chamber receptacle (3).

5. Inhaler according to any of claims 1 to 4, **characterised in that** a component, in particular a filter (9), is arranged in the inhalation channel (10) for dispersing particles.

6. Inhaler according to any of claims 1 to 5, **characterised in that** the chamber (4) has a connecting piece (8), on the side thereof which is opposite the inhalation channel (10), for coupling to the air intake opening in the casing.

7. Inhaler according to claim 2, **characterised in that** the chamber (4) is arranged in a chamber receptacle (3) and the chamber (4) has two mutually spaced holes (14) on the periphery thereof, in which holes pins (11) engage in order to pierce the end of the capsule in each case, the pins (11) being moveably mounted in the chamber receptacle (3) and able to be acted on by an actuation element (13).

8. Inhaler according to claim 2, **characterised in that** the chamber (4) is designed for single use and contains the capsule, the capsule being non-removably accommodated in the chamber (4) by the manufacturer.

9. Inhaler according to any of claims 1 to 8, **characterised in that** the chamber (4) is arranged in a chamber receptacle (3), and the casing and the chamber receptacle (3) each have at least one region made from a transparent plastics material, in particular a window, at least in the region of the chamber (4).

## Revendications

1. Inhalateur, en particulier inhalateur à poudre, pour l'administration d'un médicament sous forme de substances, de formulations de substances ou de mélanges de substances inhalables avec un boîtier, qui présente une chambre (4) pour la réception des substances, dans lequel la chambre (4) est réalisée avec une section transversale sensiblement cylindrique, dans lequel le côté frontal dirigé en direction d'un embout (2) est relié à un canal d'inhalation (10) conduisant à l'embout (2) et le côté frontal opposé à l'embout (2) est en relation active avec une ouverture d'entrée d'air du boîtier,
dans lequel la chambre (4) est agencée de manière remplaçable dans le boîtier et la chambre (4) est reliée de manière inamovible au canal d'inhalation (10) et est remplaçable conjointement avec le canal d'inhalation (10), dans lequel la chambre (4) est agencée dans un logement de chambre (3),
**caractérisé en ce que** la chambre (4) est fixée au moyen d'une liaison rainure-languette (5) dans le logement de chambre (3) en face de l'embout (2).

2. Inhalateur selon la revendication 1, **caractérisé en ce que** la chambre (4) est réalisée pour la réception d'une capsule recevant le médicament.

3. Inhalateur selon l'une quelconque des revendications 1 ou 2, **caractérisé en ce que** la chambre (4) est maintenue dans une position définie au moyen d'un codage d'enfichage.

4. Inhalateur selon l'une quelconque des revendications 1 à 3, **caractérisé en ce qu'**une butée est prévue dans le logement de chambre (3) pour la position finale insérée de la chambre (4).

5. Inhalateur selon l'une quelconque des revendications 1 à 4, **caractérisé en ce qu'**un composant, en particulier un tamis (9), est agencé dans le canal d'inhalation (10) pour la dispersion de particules.

6. Inhalateur selon l'une quelconque des revendications 1 à 5, **caractérisé en ce que** la chambre (4) présente sur le côté frontal opposé au canal d'inhalation (10), une tubulure (8) pour le couplage avec l'ouverture d'entrée d'air du boîtier.

7. Inhalateur selon la revendication 2, **caractérisé en ce que** la chambre (4) est agencée dans un logement de chambre (3) et la chambre (4) présente sur sa périphérie deux alésages (14) espacés l'un par rapport à l'autre, dans lesquels des aiguilles (11) entrent en prise pour le piquage respectivement côté extrémité de la capsule, dans lequel les aiguilles (11) sont logées de manière mobile dans le logement de chambre (3) et sont actionnables au moyen d'un élément d'actionnement (13).

8. Inhalateur selon la revendication 2, **caractérisé en ce que** la chambre (4) est réalisée pour une utilisation unique et contient la capsule, dans lequel la capsule est logée côté fabricant dans la chambre (4) de manière non retirable.

9. Inhalateur selon l'une quelconque des revendications 1 à 8, **caractérisé en ce que** la chambre (4) est agencée dans un logement de chambre (3) et le boîtier et le logement de chambre (3) présentent au moins dans la zone de la chambre (4) respectivement au moins une zone réalisée en un plastique transparent, en particulier une fenêtre.
